# EUROPEAN PATENT SPECIFICATION

(11) **EP 0 412 612 B1**
(45) Date of publication and mention of the grant of the patent: **29.01.1997**
(21) Application number: 90202134.4
(22) Date of filing: 07.08.1990
(51) Int. Cl.: C07C 209/16, C07C 209/22, C07D 295/02, C07D 295/12

(54) **Process of making polyalkylene polyamines**
Verfahren zur Herstellung von Polyalkylenpolyaminen
Procédé pour la préparation de polyalkylène polyamines

(30) Priority: 08.08.1989 US 390708
(43) Date of publication of application: 13.02.1991
(73) Proprietor: UNION CARBIDE CHEMICALS AND PLASTICS COMPANY, INC., Danbury Connecticut 06817-0001 (US)
(72) Inventor: King, Stephen Wayne, Scott Depot, West Virginia 25560 (US); Doumaux, Arthur Roy, Jr., Charleston, West Virginia 25314 (US); Schreck, David James, Cross Lane, West Virginia 25313 (US)
(74) Representative: Smulders, Theodorus A.H.J., Ir.

(56) References cited:
- EP-A- 0 228 898
- EP-A- 0 261 773
- EP-A- 0 312 253
- EP-A- 0 375 257
- WO-A-90/03963
- GB-A- 2 147 896
- US-A- 2 389 500

## Description

This invention relates to a process for making polyalkylene polyamines by condensing an amino compound in the presence of a tungsten and a Group IVB metal-containing condensation catalyst.

More in particular, the invention relates to a process providing an alkyleneamines producers composition rich in higher polyalkylene polyamines such as triethylenetetramine (TETA), tetraethylenepentamine (TEPA) and pentaethylenehexamine (PEHA), without generating large amounts of cyclics.

### Background of the Invention

There is a substantial body of literature directed to the use of various acid catalysts to effect intramolecular and intermolecular condensation of amino compounds. U.S. Patent No. 2,073,671 and U.S. Patent No. 2,467,205 constitute early prior work on the use of acid condensation. catalysts to condense amino compounds. U.S. Patent No. 2,073,671 discusses, in a general fashion, the catalytic intermolecular condensation of alcohols and amines or ammonia using the same phosphate catalysts later favored by U.S. Patent No. 2,467,205 for the intramolecular condensation of amines. The two patents are not in harmony over the use of other materials as catalysts. To illustrate this point, U.S. Patent No. 2,073,671 states:
"Alumina, thoria, blue oxide of tungsten, titania, chromic oxide, blue oxide of molybdenum and zirconia have been mentioned in the literature for use as catalysts in carrying out these reactions but their effectiveness is so low that no practical application has been made of their use.",
whereas U.S. Patent No. 2,467,205 in describing the self-condensation of ethylenediamine (EDA) under vapor phase conditions, to initially produce ethyleneamines, but after recycle, eventually generates piperazine through multistep condensation reactions, followed by deamination, recommends "dehydration catalysts" which are thereafter characterized as
"silica gel, titania gel, alumina, thoria, boron phosphate, aluminum phosphate, and the like."

U.S. Patent No 2,073,671 describes the condensation catalyst in the following terms:
"...a heated catalyst or contact mass containing phosphorus and especially one or more of the oxygen acids of phosphorus, their anhydrides, their polymers, and their salts; for example, orthophosphoric acid, metaphosphoric acid, pyrophosphoric acid, phosphorous pentoxide, dimetaphosphoric acid, trimetaphosphoric acid, primary ammonium phosphate, secondary ammonium phosphate, normal ammonium phosphate, ammonium metaphosphate, secondary ammonium pyrophosphate, normal ammonium pyrophosphate, aluminum phosphate, aluminum acid phosphate and mixtures of two or more of such materials."
whereas U.S. Patent No. 2,467,205 describes one of the preferred catalysts as "basic aluminum phosphate".

U.S. Patent No. 2,454,404 describes the "catalytic deamination of alkylene polyamines" by reacting diethylenetriamine (DETA) vapor over solid catalysts such as activated alumina, bauxite, certain aluminum silicates such as kaolin and oxides of thorium, titanium and zirconium.

U.S. Patent Nos. 2,073,671 and 2,467,205 demonstrate a common experience in using aluminum phosphate as a condensation catalyst to produce aliphatic amines, and U.S. Patent Nos. 2,454,404 and 2,467,205 contemplate the other solid catalysts for deamination of amines to make heterocyclic noncyclic amines. In general, the reaction conditions under which deamination to effect cyclization occurs are more severe than those employed for condensation to generate noncyclic molecules, all other factors being comparable.

U.S. Patent Nos. 4,540,822, 4,584,406 and 4,588,842 depict the use of Group IVB metal oxides as supports for phosphorus catalysts used to effect the condensation of amino compounds with alkanolamines.

U.S. Patent No. 4,683,335 describes the use of tungstophosphoric acid, molybdophosphoric acid or mixtures deposited on titania as catalysts for the condensation of amines and alkanolamines to make polyalkylenepolyamines.

U.S. Patent Nos. 4,314,083, 4,316,840, 4,362,886 and 4,394,524 disclose the use of certain metal sulfates as useful catalysts for the condensation of alkanolamine and an amino compound. No distinction is made between the sulfur compounds in respect to catalytic efficacy. Sulfuric acid is as good as any metal sulfate, and all metal sulfates are treated as equivalents. At column 8 of U.S. Patent No. 4,314,083, it is noted that boron sulfate "gave extremely high selectivity at a low level" of EDA. However, selectivity in general was shown to increase with an increase of EDA relative to MEA in the feed. The only specific metal sulfates disclosed in the patents are antimony sulfate, beryllium sulfate, iron sulfate and aluminum sulfate.

In the typical case of the manufacture of alkyleneamines, mixtures with other alkyleneamines (including a variety of polyalkylenepolyamines and cyclic alkylenepolyamines) are formed. The same holds true when the object of the process is to produce polyalkylenepolyamines whether acyclic or cyclic, in that a variety of amino compounds are also formed. Each of these cyclic and acyclic alkyleneamines can be isolated from the mixture.

The acid catalyzed condensation reaction involving the reaction of an alkanolamine with an amino compound in the presence of an acidic catalyst is believed to proceed through the mechanism of esterifying free surface hydroxyl groups on the acid catalyst with the alkanolamine and/or by protonating the alkanolamine in the presence of the acid catalyst, followed by loss of water and amine condensation of the ester or the hydrated species, as the case may be, to form the alkyleneamine. Illustrative prior art directed primarily to the cyclic polyalkylenepolyamines (heterocyclic polyamines), but not necessarily limited to the aforementioned acid condensation reaction, are: U.S. Patent Nos. 2,937,176, 2,977,363, 2,977,364, 2,985,658, 3,056,788, 3,231,573, 3,167,555, 3,242,183, 3,297,701, 3,172,891, 3,369,019, 3,342,820, 3,956,329, 4,017,494, 4,092,316, 4,182,864, 4,405,784 and 4,514,567; European Patent Applications 0 069 322, 0 111 928 and 0 158 319; East German Patent No. 206,896; Japanese Patent Publication No. 51-141895; and French Patent No. 1,381,243. The evolution of the art to the use of the acid catalyzed condensation reaction to generate acyclic alkyleneamines, particularly acyclic polyalkylenepolyamines, as the predominant products stemmed from the initial disclosure in U.S. Patent No. 4,036,881, though earlier patent literature fairly well characterized such an effect without labeling it so, see U.S. Patent No. 2,467,205, supra. The acid catalysts are phosphorus compounds and the reaction is carried out in the liquid phase. The trend in this catalyst direction was early set as demonstrated by U.S. Patent Nos. 2,073,671 and 2,467,205, supra. A modification of this route includes the addition of ammonia to the reaction, see, for example, U.S. Patent No. 4,394,524 and U.S. Patent No. 4,463,193 for the purpose of converting alkanolamine such as MEA in situ to alkylene amine such as EDA by reaction with ammonia, and the EDA, is in situ reacted with MEA according to the process of U.S. Patent No. 4,036,881 to form alkyleneamines.

A summary of the prior art employing acid catalysts for making alkyleneamines is set forth in Table 1 below.

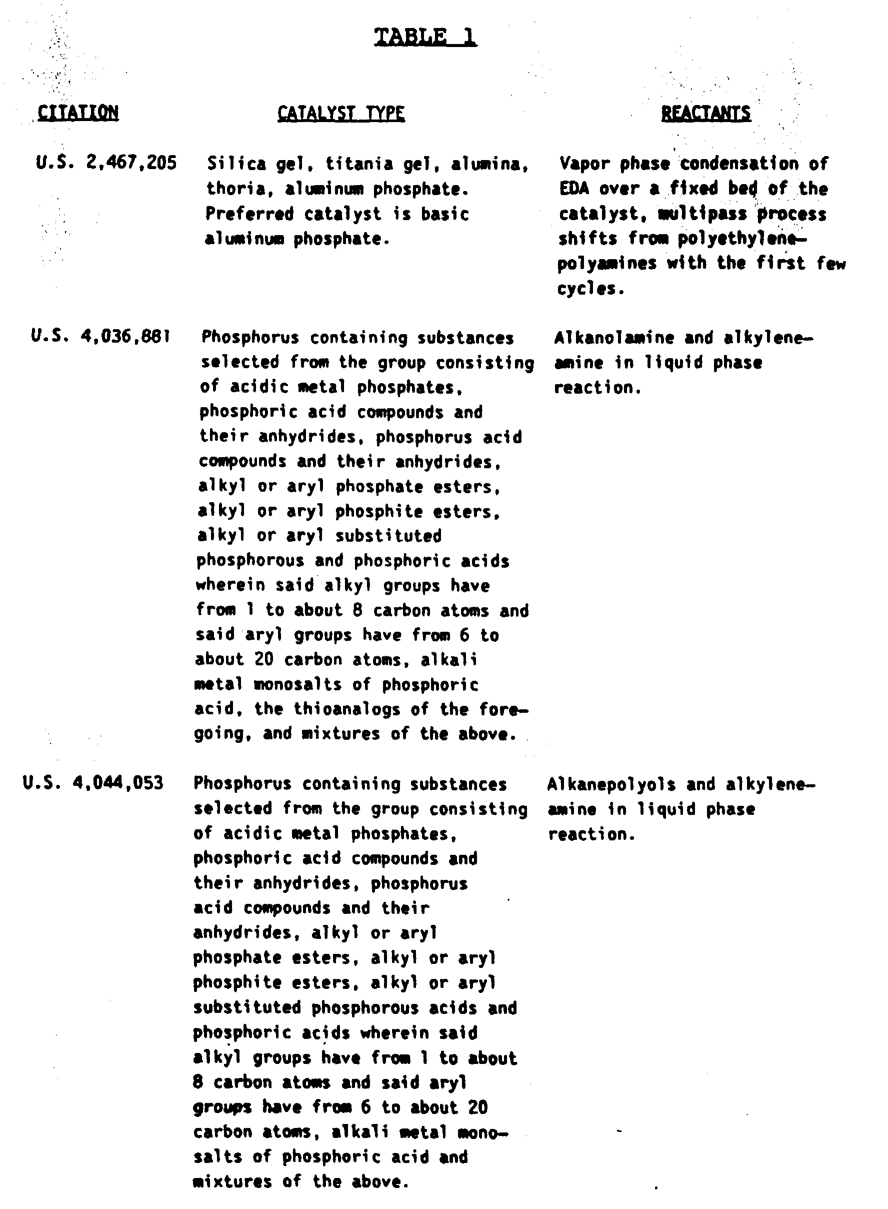

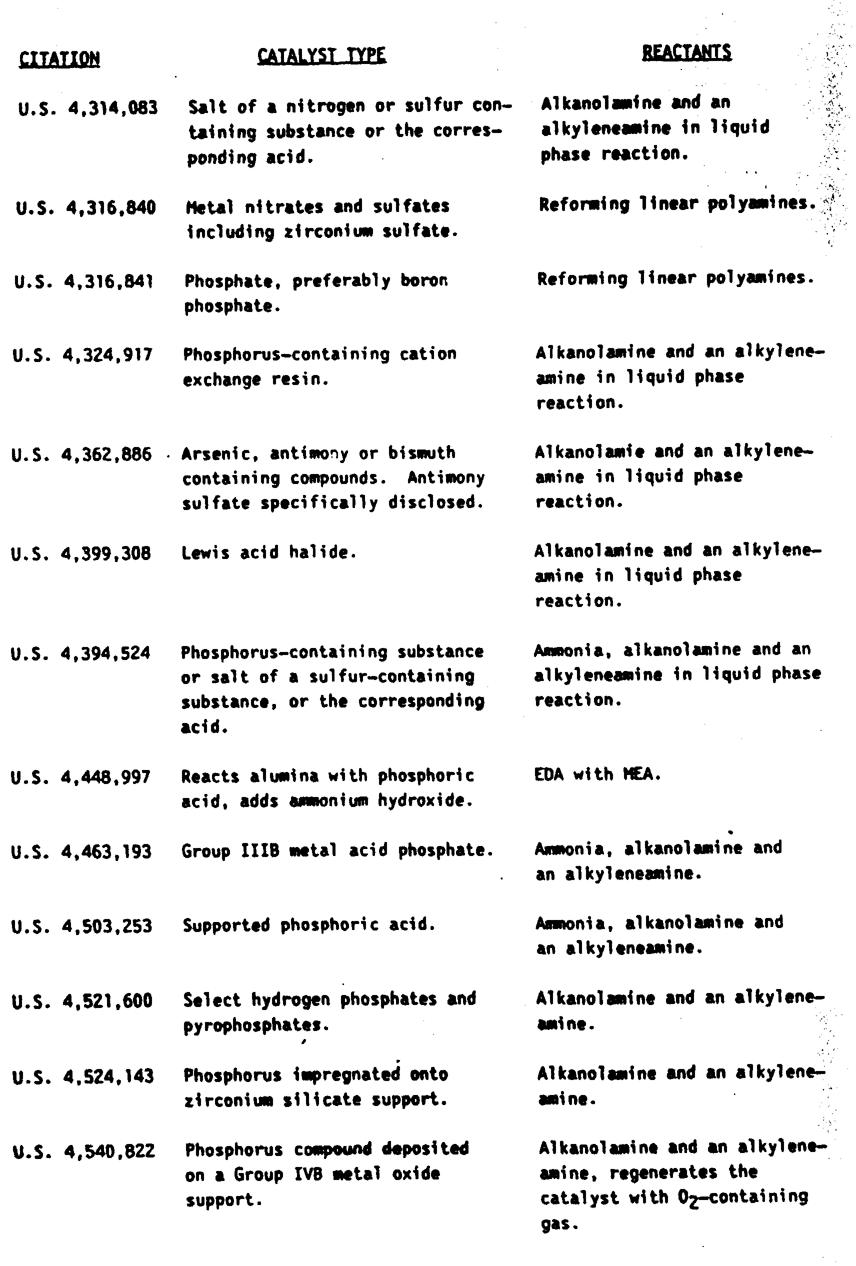

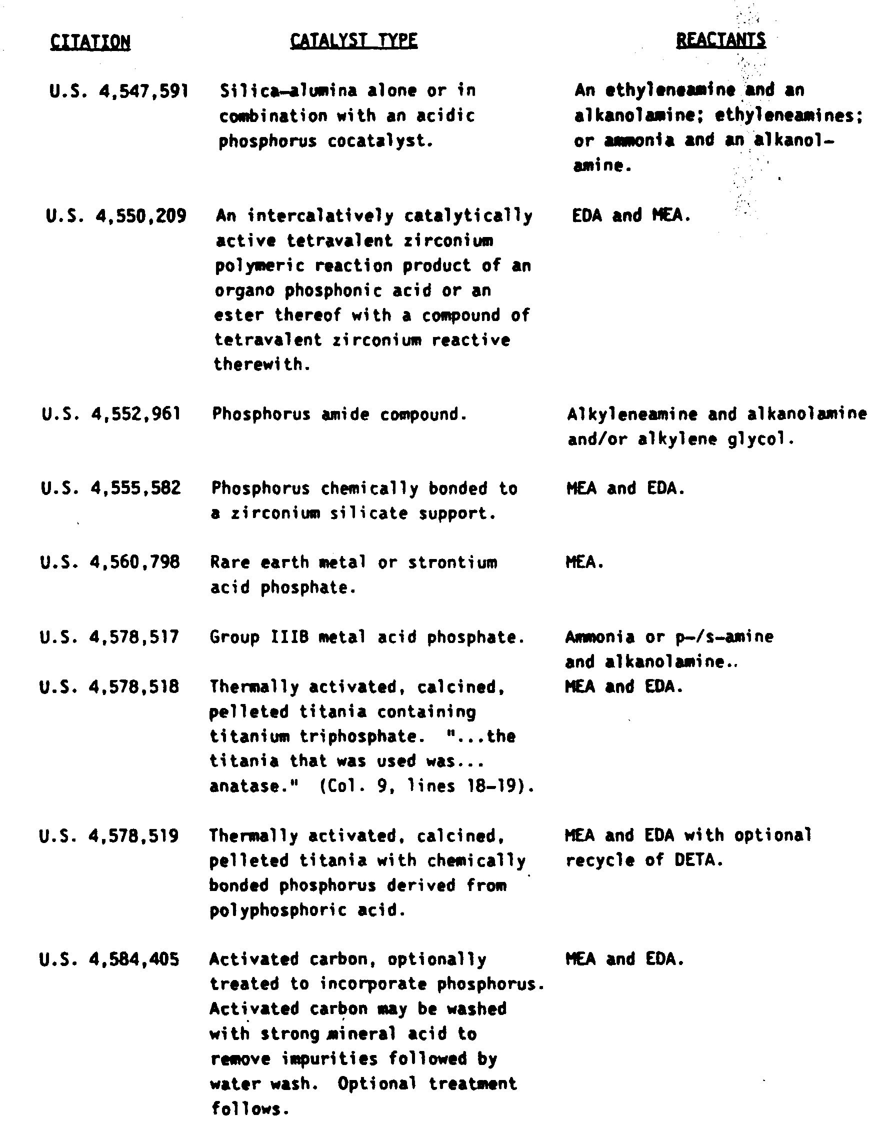

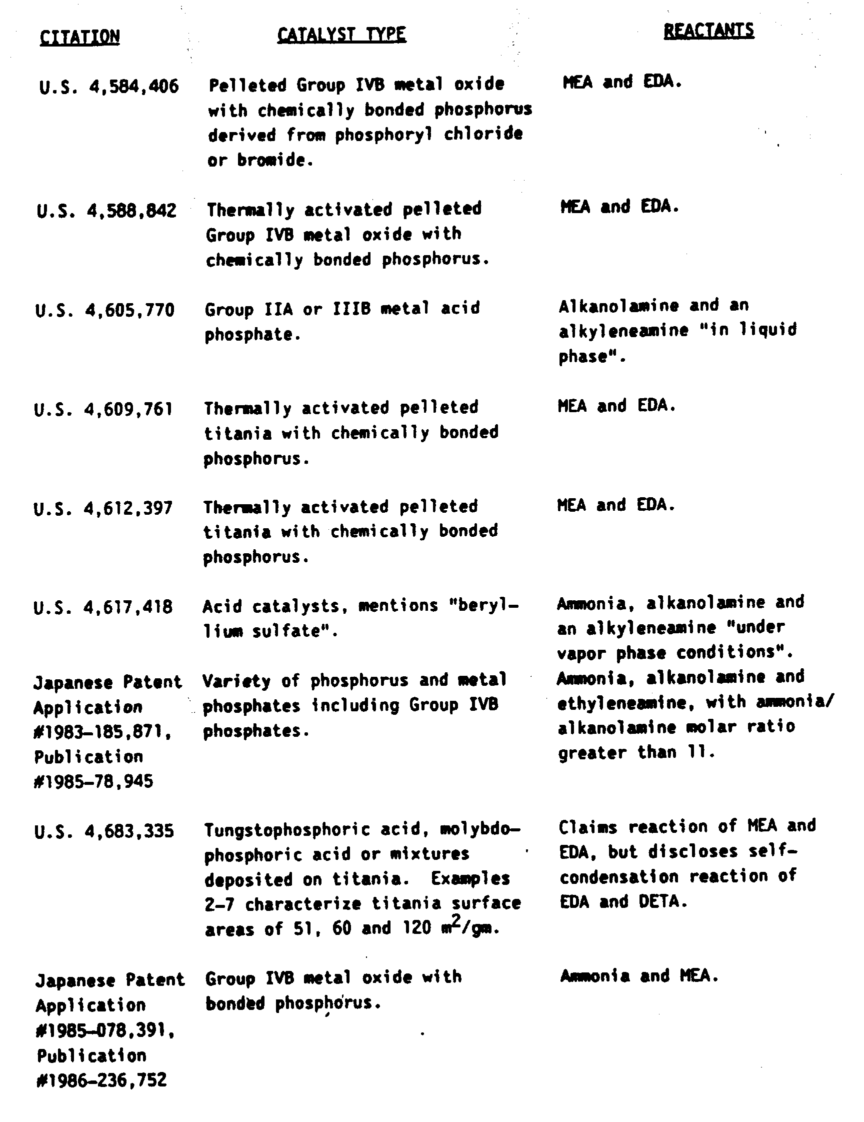

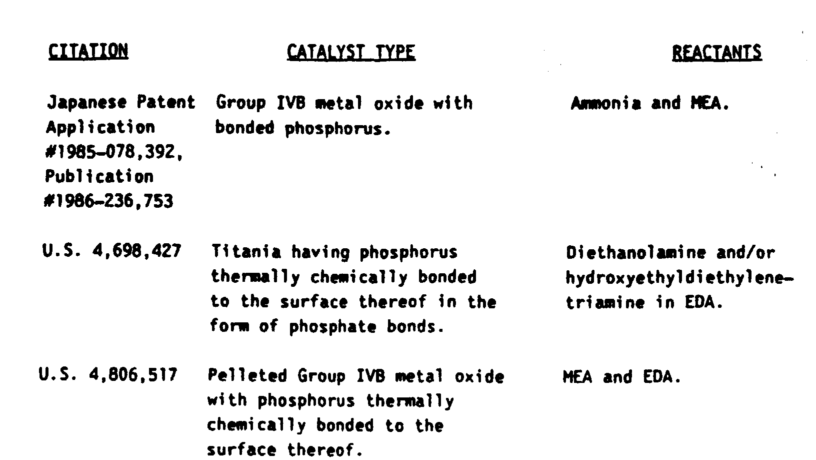

The market demand for higher polyalkylene polyamines such as TETA, TEPA and PEHA has been progressively increasing in recent years. These higher polyalkylene polyamines are desirable co-products with DETA. It would be desirable to satisfy the existing demand from a cost standpoint by modifying slightly the commercial processes directed to the manufacture of DETA from the reaction of MEA and EDA or other suitable starting raw materials such as DETA and AEEA, to the production of TETA, TEPA and PEHA as major products.

Furthermore, reference is made to European patent applications 0 261 773 and 0 375 257. These applications relate to preparation processes for predominantly linear polyethylene polyamines by using tungstophosphoric acid and tungstosilicic acid, respectively, on titania.

It would be desirable to have continuously produced compositions, generated by the reaction of MEA and EDA or other suitable starting raw materials such as DETA and AEEA over a fixed bed of a condensation catalyst under commercial conditions, that are rich in TETA, TEPA and PEHA, and that are not disproporationately high in PIP and other cyclics.

It would be very beneficial to have a process which increases one's ability to generate the manufacture of desirable higher polyalkylene polyamine products such as TETA, TEPA and PEHA without generating large amounts of cyclic alkylenepolyamine products. In addition, it would also be desirable to have a process with raw material flexibility which provides the potential to control congener distribution, linear to cyclic selectivity and linear to branched selectivity of the higher polyalkylene polyamines products. As used herein, congener distribution refers to polyalkylene polyamines containing the same number of nitrogen atoms but not necessarily having the same molecular weight or structure.

The prior art catalysts which are acidic in nature, such as the catalysts used in EP-A-0 261 773 and 0 375 257, lead to stability problems in amines reaction media when utilized over an extended period of time. If used on supports, these prior art catalysts may suffer from teaching problems. These problems will be reverted to in more detail hereinbelow. The process of the present invention does not have the above-mentioned problems related with the prior art catalysts and provides the above advantageous features.

In detail, the present invention relates to a process of making polyalkylene polyamines without generating large amounts of cyclics which process comprises condensing, in the absence of hydrogen atoms as a reactant, (i) an alkanolamine with an alkylene amine or (ii) an alkanolamine with itself or another alkanolamine, optionally in the presence of ammonia, under condensation conditions in the presence of a condensation catalyst obtained by calcining a reaction mixture comprising ammonium metatungstate and one or more Group IVB metal oxides.

The calcined ammonium metatungstate-containing condensation catalysts used herein contain sufficient residual bound hydroxyl groups or other groupings which renders catalyst formation possible by loss of water or its chemical equivalent such as ammonium hydroxide.

While using the process of the invention a continuously generated alkyleneamines producers composition is obtained comprising, based on 100 percent of the weight of the composition and exclusive of any water and/or ammonia present,
a) greater than about 3.0 weight percent of the combination of TETA and TEPA,
b) greater than about 0.1 weight percent of TEPA,
c) greater than about 3.0 weight percent of TETA,
d) less than about 90.0 weight percent of DETA and/or EDA,
e) less than about 90.0 weight percent of MEA and/or AEEA,
f) less than about 12.5 weight percent of the combination of PIP and AEP,
g) less than about 15.0 weight percent of other polyalkylene polyamines,
h) a TETA + TAEA to PIP + AEP + PEEDA + DAEP + DPE weight ratio of greater than about 0.5,
i) a TEPA + AETAEA to PIP + AEP + PEEDA + DAEP + DPE + AEPEEDA + iAEPEEDA + AEDAEP + AEDPE + BPEA weight ratio of greater than about 0.5,
j) a TETA to TAEA weight ratio of greater than about 4.0, and
k) a TEPA to AETAEA weight ratio of greater than about 1.0.

As used herein, the term "amino compound" embraces ammonia and any compound containing nitrogen to which is bonded an active hydrogen. Also, for purposes of this invention, the term "oxide" embraces oxides, hydroxides and/or mixtures thereof.

For purposes of this invention, the chemical elements are identified in accordance with the Periodic Table of the Elements, CAS version, Handbook of Chemistry and Physics, 67th Ed., 1986-87, inside cover. Also, for purposes of this invention, Group IIIB metal oxides embraces the lanthanides and actinides.

### Detailed Description

The higher polyalkylene polyamines such as TETA, TEPA and PEHA are very useful commercial products for a variety of applications including fuel oil additives, corrosion inhibitors, fabric softeners, fungicides and others. As indicated above, there is lacking a commercial process for the manufacture of enhanced quantities of TETA, TEPA and PEHA especially as significant products of reaction. There is thus a need for the ability to commercially generate larger production quantities of TETA, TEPA and PEHA and that is the direction of this invention. The process of this invention provides for the reaction of MEA and DETA or other suitable starting raw materials such as EDA and AEEA to produce in a continuous manner a reaction product mixture, termed herein an "alkyleneamines producers composition", in which TETA, TEPA and PEHA are principal products of the reaction.

The process of this invention is distinctive insofar as it achieves the generation of high concentrations of TETA, TEPA and PEHA in a manner which can be suitably employed in a commercial process, particularly a continuous process, for the manufacture of alkyleneamines. In particular, the process of this invention allows the production of TETA, TEPA and PEHA in relatively high yields without generating large amounts of cyclic polyalkylene polyamine products. The process of this invention provides starting raw material flexibility thereby allowing the potential to control congener distribution, linear to cyclic selectivity and linear to branched selectivity of the higher polyalkylene polyamine products.

As indicated above, this invention relates to a process of making polyalkylene polyamines which comprises condensing an amino compound in the presence of a condensation catalyst obtained by calcining a reaction mixture comparising ammonium metatungstate and one or more Group IVB metal oxides.

As also indicated above, while using the process of the invention a continuously generated polyalkylene polyamines producers composition is obtained, based on 100 percent of the weight of the composition and exclusive of any water and/or ammonia present,
a) greater than about 3.0 weight percent of the combination of TETA and TEPA,
b) greater than about 0.1 weight percent of TEPA,
c) greater than about 3.0 weight percent of TETA,
d) less than about 90.0 weight percent of DETA and/or EDA,
e) less than about 90.0 weight percent of MEA and/or AEEA,
f) less than about 12.5 weight percent of the combination of PIP and AEP,
g) less than about 15.0 weight percent of other polyalkylene polyamines,
h) a TETA + TAEA to PIP + AEP + PEEDA + DAEP + DPE weight ratio of greater than about 0.5,
i) a TEPA + AETAEA to PIP + AEP + PEEDA + DAEP + DPE + AEPEEDA + iAEPEEDA + AEDAEP + AEDPE + BPEA weight ratio of greater than about 0.5,
j) a TETA to TAEA weight ratio of greater than about 4.0, and
k) a TEPA to AETAEA weight ratio of greater than about 1.0.

The alkyleneamines producers composition can be subjected to conventional separation techniques for recovering the individual components of the composition. Such techniques are well known in the art and include, for example, distillation.

This invention contemplates the catalyzed condensation by (i) condensation of an alkanolamine to an alkyleneamine having a lower molecular weight, and (ii) condensation of an alkanolamine with itself or with one or more other alkanolamines to an amine having a lower, same or higher molecular weight than the reactants, in the presence of a condensation catalyst obtained by calcining a reaction mixture comprising ammonium metatungstate a Group IVB metal oxide.

While not wishing to be bound to any particular theory, it is believed that those calcined ammonium metatungstate and Group IVB metal-containing substances encompassed within the scope of this invention possessing ionic character and/or ion exchange capacity exhibit desired catalytic activity and provide desired product selectivity. It is believed to be desirable that such calcined ammonium metatungstate and Group IVB metal-containing substances possessing ionic character and/or ion exchange capacity be formed in situ in order to provide desired catalytic activity and product selectivity. In such instances, the catalyst preparation conditions or reaction conditions should allow for the formation calcined ammonium metatungstate and Group IVB metal-containing substances possessing ionic character and/or ion exchange capacity. Mixtures of calcined ammonium metatungstate and Group IVB metal-containing substances possessing ionic character and/or ion exchange capacity with Group IVB metal-containing substances having other than ionic character and/or ion exchange capacity are believed to exhibit desired catalytic activity and provide desired product selectivity.

The calcined ammonium metatungstate Group IVB metal-containing condensation catalyst can be prepared by conventional methods known in the art.

The level of activity of the condensation catalyst of the invention is that level which of itself makes the catalyst at least as active in the condensation of amines as, for example, is phosphoric acid on an equivalent basis. Preferably, supported calcined ammonium metatungstate-containing condensation catalysts should have a surface area greater than 70 m²/gm to as high as 260 m²/gm or greater depending upon which metal oxide described below that is employed. In the case of calcined ammonium metatungstate-containing substances in association with titanium oxides, the surface area should be greater than 140 m²/gm to as high as 260 m²/gm, more preferably, greater than 160 m²/gm to as high as 260 m²/gm, determined according to the single point N2 method. In the case of calcined ammonium metatungstate-containing substances in association with zirconia oxides, the surface area should be greater than 70 m²/gm to as high as 150 m²/gm, more preferably, greater than 90 m²/gm to as high as 135 m²/gm, determined according to the single point N₂ method. It is appreaciated that the metal oxides described below which can be used in association with the calcined ammonium metatungstate-containing condensation catalyst and the performance moderators described below can affect the surface area of the condensation catalyst used in the process of the invention. While surface areas described above may be preferred, for purposes of this invention, the surface area of the used condensation catalyst should be sufficient to contribute to product selectivity, catalytic activity and/or mechanical or dimensional strength of the catalyst.

The condensation catalysts used in accordance with the present invention are associated with one or more Group IVB metal oxides. Preferred metal oxides are amphoteric or slightly acidic or slightly basic. Group IVB metal oxides which may be utilized in the preparation of the condensation catalyst used in the process of the invention include, for example, one or more oxides of titanium, zirconium and hafnium.

For mixed metal oxides in which at least one of the metals is titanium, suitable metals in association with titanium may include, for example, one or more of the following: Group IIIB metals such as scandium, yttrium and lanthanum including the lanthanides, Group VB metals such as niobium and tantalum, other Group VIB metals such as chromium, molybdenum and tungsten, Group VIII metals such as iron, cobalt and nickel, Group IIB metals such as zinc and cadmium, Group IIIA metals such as boron, aluminum, gallium and indium, Group IVA metals such as silicon, germanium, tin and lead, Group VA metals such as arsenic, antimony and bismuth, and Group IVB metals such as zirconium and hafnium. For mixed metal oxides in which at least one of the metals is zirconium, suitable metals in association with zirconium may include, for example, one or more of the following: Group IVA metals such as silicon, germanium, tin and lead, Group VB metals such as niobium and tantalum, and other Group VIB metals such as chromium, molybdenum and tungsten. The virtue of these metal oxides is that they demonstrate higher mechanical strength than calcined ammonium metatungstate per se and can contribute to product selectivity and catalytic activity.

Illustrative of mixed metal oxides which may be used in association with the calcined condensation catalyst used in the process of the invention include, for example, TiO₂-SiO₂, TiO₂-Al₂O₃, TiO₂-CdO, TiO₂-Bi₂O₃, TiO₂-Sb₂O₅, TiO₂-SnO₂, TiO₂-ZrO₂, TiO₂-BeO, TiO₂-MgO, TiO₂-CaO, TiO₂-SrO, TiO₂-ZnO, TiO₂-Ga₂O₃, TiO₂-Y₂O₃, TiO₂-La₂O₃, TiO₂-MoO₃, TiO₂-Mn₂O₃, TiO₂-Fe₂O₃, TiO₂-Co₃O₄, TiO₂-WO₃, TiO₂-V₂O₅, TiO₂-Cr₂O₃, TiO₂-ThO₂, TiO₂-Na₂O, TiO₂-BaO, TiO₂-CaO, TiO₂-HfO₂, TiO₂-Li₂O, TiO₂-Nb₂O₅, TiO₂-Ta₂O₅, TiO₂-Gd₂O₃, TiO₂-Lu₂O₃, TiO₂-Yb₂O₃, TiO₂-CeO₂, TiO₂-Sc₂O₃, TiO₂-PbO, TiO₂-NiO, TiO₂-CUO, TiO₂-CoO, TiO₂-B₂O₃, ZrO₂-SiO₂, ZrO₂-Al₂O₃, ZrO₂-SnO, ZrO₂-PbO, ZrO₂-Nb₂O₅, ZrO₂-Ta₂O₅, ZrO₂-Cr₂O₃, ZrO₂-MoO₃, ZrO₂-WO₃, ZrO₂-TiO₂, ZrO₂-HfO₂, TiO₂-SiO₂-Al₂O₃, TiO₂-SiO₂-ZnO, TiO₂-SiO₂-ZrO₂, TiO₂-SiB₂-CuO, TiO₂-SiO₂-MgO, TiO₂-SiO₂-Fe₂O₃, TiO₂-SiO₂-B₂O₃, TiO₂-SiO₂-WO₃, TiO₂-SiO₂-Na₂O, TiO₂-SiO₂-MgO, TiO₂-SiO₂-La₂O₃, TiO₂-SiO₂-Nb₂O₅, TiO₂-SiO₂-Mn₂O₃, TiO₂-SiO₂-Co₃O₄, TiO₂-SiO₂-NiO, TiO₂-SiO₂-PbO, TiO₂-SiO₂-Bi₂O₃, TiO₂-Al₂O₃-ZnO, TiO₂-Al₂O₃-ZrO₂, TiO₂-Al₂O₃-Fe₂O₃, TiO₂-Al₂O₃-WO₃, TiO₂-Al₂O₃-La₂O₃, TiO₂-Al₂O₃-Co₃O₄, ZrO₂-SiO₂-Al₂O₃-, ZrO₂-SiO₂-SnO, ZrO₂-SiO₂-Nb₂O₅, ZrO₂-SiO₂-WO₃, ZrO₂-SiO₂-TiO₂, ZrO₂-SiO₂-MoO₃, ZrO₂-SiO₂-HfO₂, ZrO₂-SiO₂-Ta₂O₅, ZrO₂-Al₂O₃-SiO₂, ZrO₂-Al₂O₃-PbO, ZrO₂-Al₂O₃-Nb₂O₅, ZrO₂-Al₂O₃-WO₃, ZrO₂-Al₂O₃-TiO₂, ZrO₂-Al₂O₃-MoO₃, ZrO₂-HfO₂ Al₂O₃, ZrO₂-HfO₂-TiO₂. Other suitable mixed metal oxide catalysts embraced within the scope of this invention are disclosed by Tanabe et al., Bulletin of the Chemical Society of Japan, Vol. 47(5), pp. 1064-1066 (1974).

The metal oxides described herein which can be used in the condensation catalyst which is used in the process of the present invention may contribute to product selectivity and/or catalytic activity of the reaction and/or stability of the catalyst. The catalyst structure can comprise from 0 to 90 percent or greater by weight of the metal oxide, preferably from 0 to 75 percent by weight of the metal oxide, and more preferably from 0 to 50 percent by weight of the metal oxide, the remainder being the weight of the condensation catalyst obtained by calcining a reaction mixture comprising ammonium metatungstate and one or more Group IVB metal oxides. For mixed metal oxides containing titania, higher concentrations of titania can provide very desirable product selectivities including acyclic to cyclic selectivities and linear to branched selectivities of higher polyalkylene polyamine products. As discussed hereinafter, the condensation catalyst used in the process of this invention may also contain support(s), binding agent(s) or other additives to stabilize or otherwise help in the manufacture of the catalyst.

Though the condensation catalyst used in the process of the invention provides sufficient activity to effect the condensation reaction, certain combinations of reactants and/or product formation may be benefited by treating the catalyst with a catalyst moderator, hereinafter termed "performance moderator." Catalyst moderators are widely used to control the performance of catalysts in areas of selectivity to certain products and the repression of a catalyst's proclivity to generate a broad range of reaction products. A range of suitable materials may impact the condensation catalysts obtained by calcining a reaction mixture comprising ammonium metatungstate and one or more Group IVB metal oxides in the variety of reaction products. The performance moderator may be any material which impacts the condensation catalyst's selection of reaction products or which changes the proportion of any one or more of the reaction products which this condensation catalyst generates at comparable processing conditions. In addition to contributing to product selectivity, the performance moderator may be any material which contributes to catalytic activity and/or catalyst stability (mechanical or dimensional strength).

An illustrative performance moderator is a mineral acid or a compound derived from a mineral acid. Suitable for use as performance moderators are one or more phosphoric acid or a salt of phosphoric acid, hydrogen fluoride, hydrofluoric acid or a fluoride salt, sulfuric acid or a salt of sulfuric acid. The moderator may also be organic esters of phosphoric acid or a salt of phosphoric acid, hydrogen fluoride organic complexes, hydrofluoric acid organic complexes or a fluoride salt organic complexes, organic esters of sulfuric acid or a salt of sulfuric acid. Suitable salts of phosphoric acid include sodium dihydrogen phosphate, disodium hydrogen phosphate, sodium phosphate. Other illustrative performance moderators include the metal oxides described above which are used in the reaction mixture which provides - after calcination the condensation catalyst to be used in the process of the present invention and also metallic phosphates and polyphosphates which may or may not have a cyclic structure and whicn may or may not have a condensed structure.

The metallic phosphates and polyphosphates are preferred performance moderators for use in this invention. The metallic phosphate and polyphosphate performance moderators may or may not have a cyclic structure and may or may not have a condensed structure. Suitable metallic phosphates having a cyclic structure or an acyclic structure are disclosed in U.S. Patent Application Serial No. 390,706, filed on an even date herewith and incorporated herein by reference. Suitable metallic polyphosphates having a condensed structure are disclosed in U.S. Patent Application Serial No.390,709, filed on an even date herewith and incorporated herein by reference.

Suitable metallic phosphate and polyphosphate performance moderators can include, for example, metallic orthophosphates (PO₄ ⁻³), metallic pyrophosphates (P₂O₇ ⁻⁴), metallic polyphosphates (including tripolyphosphates (P₃O₁₀ ⁻⁵), tetrapolyphosphates (P₄O₁₃ ⁻⁶), pentapolyphosphates (P₅O₁₆ ⁻⁷) and higher polyphosphates), metallic metaphosphates (including trimetaphosphates (P₃O₉ ⁻³), tetrametaphosphates (P₄O₁₂ ⁻⁴) and other lower and higher metaphosphates) and metallic ultraphosphates (condensed phosphates containing more P₂O₅ than corresponds to the metaphosphate structure). Corresponding metallic metaphosphimates, metallic phosphoramidates and metallic amido- and imidophosphates of the above may also be used as performance moderators in accordance with this invention. Suitable metals which can be incorporated into the metallic phosphate and polyphosphate performance moderators include, for example, Group IA metals, Group IIA metals, Group IIIB metals, Group IVB metals, Group VB metals, Group VIB metals, Group VIIB metals, Group VIII metals, Group IB metals, Group IIB metals, Group IIIA metals, Group IVA metals, Group VA metals, Group VIA metals and mixtures thereof.

Illustrative of metallic orthophosphate performance moderators which may be utilized in this invention include, for example, NaH₂PO₄, KH₂PO₄, RbH₂PO₄, LiH₂PO₄, MgHPO₄, CaHPO₄, YPO₄, CePO₄, LaPO₄, ThPO₄, MnPO₄, FePO₄, BPO₄, AlPO₄, BiPO₄, Mg(H₂PO₄)₂, Ba(H₂PO₄)₂, Mg(NH₄)₂PO₄, Ca(H₂PO₄)₂, La(H₂PO₄)₃. Illustrative of metallic pyrophosphate performance moderators which may be utilized in this invention include, for example, Na₂H₂P₂O₇, K₂H₂P₂O₇, Ca₂P₂O₇, Mg₂P₂O₇, KMnP₂O₇, AgMnP₂O₇, BaMnP₂O₇, NaMnP₂O₇, KCrP₂O₇, NaCrP₂O₇, Na₄P₂O₇, K₄P₂O₇, Na₃HP₂O₇, NaH₃P₂O₇, SiP₂O₇, ZrP₂O₇, Na₆Fe₂(P₂O₇)₃, Na₈Fe₄(P₂O₇)5, Na₆Cu(P₂O₇)₂, Na₃₂Cu₁₄(P₂O₇)₁₅, Na₄Cu₁₈(P₂O₇)₅, Na₂(NH₄)₂P₂O₇, Ca(NH₄)₂P₂O₇, MgH₂P₂O₇, Mg(NH₄)₂P₂O₇. Illustrative of metallic polyphosphate performance moderators which may be utilized in this invention include, for example, NaSr₂P₃O₁₀, NaCa₂P₃O₁₀, NaNi₂P₃O₁₀, Na₅P₃O₁₀, K₅P₃O₁₀, Na₃MgP₃O₁₀, Na₃CuP₃O₁₀, Cu₅(P₃O₁₀)₂, Na₃ZnP₃O₁₀, Na₃CdP₃O₁₀, Na₆Pb(P₃O₁₀)₂, Na₃CoP₃O₁₀, K₃CoP₃O₁₀, Na₃NiP₃O₁₀, K₂(NH₄)₃P₃O₁₀, Ca(NH₄)₂P₃O₁₀, La(NH₄)₂P₃O₁₀, NaMgH₂P₃O₁₀. Illustrative of metallic metaphosphate performance moderators which may be utilized in this invention include, for example, Na₃P₃O₉, K₃P₃O₉, Ag₃P₃O₉, Na₄P₄O₁₂, K₄P₄O₁₂, Na₂HP₃O₉, Na₄Mg(P₃O₉)₂, NaSrP₃O₉, NaCaP₃O₉, NaBaP₃O₉, KBaP₃O₉, Ca₃(P₃O₉)₂, Ba(P₃O₉)₂, Na₂Ni₂(P₃O₉)₂, Na₄Ni(P₃O₉)₂, Na₄Co(P₃O₉)₂, Na₄Cd(P₃O₉)₂. Illustrative of metallic ultraphosphate performance moderators which may be utilized in this invention include, for example, CaP₄O₁₁, Ca₂P₆O₁₇,Na₈P₁₀O₂₉, Na₆P₈O₂₃, Na₂CaP₆O₁₇, Na₂P₄O₁₁, NaBaP₇O₁₈, Na₂P₈O₂₁, K₄P₆O₁₇. The preferred metallic phosphate and polyphosphate performance moderators for use in this invention include Group IA metal metaphosphates, Group IA metal dihydrogen orthophosphates and Group IA metal dihydrogen pyrophosphates, more preferably Na₃P₃O₉, NaH₂PO₄ and Na₂H₂P₂O₇. Other suitable metallic phosphate and polyphosphate performance moderators which are embraced within the scope of this invention are disclosed by Van Wazer, J.R., Phosphorus and Its Compounds, Vol. 1, Interscience Publishers, Inc., New York (1958).

The metal oxides described hereinabove which can be used in association with the condensation catalyst obtained by calcining a reaction mixture comprising ammonium metatungstate and one or more Group IVB metal oxides can also be used as performance moderators in accordance with this invention. The metal oxides can contribute to product selectivity, catalytic activity and/or catalyst stability (mechanical strength).

A variety of conventional phosphorus-containing substances may be suitable for use as performance moderators in this invention. The conventional substances should be capable of functioning as a performance moderator. Illustrative of conventional phosphorus-containing substances may include, for example, those disclosed in U.S. Patent No. 4,036,881, U.S. Patent No. 4,806,517, U.S. Patent No. 4,617,418, U.S. Patent No. 4,720,588, U.S. Patent No. 4,394,524, U.S. Patent No. 4,540,822, U.S. Patent No. 4,588,842, U.S. Patent No. 4,605,770, U.S. Patent No. 4,683,335, U.S. Patent No. 4,316,841, U.S. Patent No. 4,463,193, U.S. Patent No. 4,503,253, U.S. Patent No. 4,560,798 and U.S. Patent No. 4,578,517.

Suitable conventional phosphorus-containing substances which may be employed as performance moderators in this invention include acidic metal phosphates, phosphoric acid compounds and their anhydrides, phosphorous acid compounds and their anhydrides, alkyl or aryl phosphate esters, alkyl or aryl phosphite esters, alkyl or aryl substituted phosphorous acids and phosphoric acids, alkali metal monosalts of phosphoric acid, the thioanalogs of the foregoing, and mixtures of any of the above.

The amount of the performance moderator of the mineral acid type used with condenstation catalyst used in the process of the invention is not narrowly critical. Generally, the amount does not exceed 25 weight percent of the weight of the catalyst. As a rule, it is desirable to use at least 0.01 weight percent of the weight of the catalyst. Preferably, the amount of performance moderator, when used, will range from 0.2 to 10 weight percent of the weight of the catalyst. Most preferably, the amount of performance moderator, when used, will range from 0.5 to 5 weight percent of the weight of the catalyst.

The amount of performance moderator other than the mineral acid type used with the containing catalyst used in the process of the invention is not narrowly critical. Generally, the amount does not exceed 90 weight percent of the weight of the catalyst. The amount of performance moderator can range from 0 to 90 or greater weight percent of the weight of the catalyst, preferably from 0 to 75 weight percent of the weight of the catalyst, and more preferably from 0 to 50 weight percent of the weight of the catalyst. Most preferably, the amount of performance moderator, when used, will range from 0.5 to 25 weight percent of the weight of the catalyst.

This invention also embraces the use of vicinal di(hetero)alkylene organometalates in the preparation of amines. Suitable vicinal di(hetero)alkylene organometalates are disclosed in EP-A-0 412 615.

The performance moderator can be provided to the condensation catalyst obtained by calcining a reaction mixture comprising ammonium metatungstate and one or more Group IVB metal oxides by conventional procedures known in the art. For example, the performance moderator can be provided to the catalyst by impregnating particles or monolithic structures comprising the condensation catalyst obtained by calcining a reaction mixture comprising ammonium metatungstate and one or more Group IVB metal oxides with liquid comprising the performance moderator. This is a well known procedure in the art for incorporating additives to a solid support material. The condensation catalyst used in the process of the invention may be utilized as solid powders or as fused, bonded or compressed solid pellets, or larger structures in association with the one or more metal oxides, or as coated, fused, bonded or compressed solid pellets, or larger structures, composited with one or more support materials, in association with one or more metal oxides. These solid structures may be treated with the performance moderator by mixing a liquid body of the performance moderator with the solid structure. For example, the condensation catalyst solids may be slurried in the performance moderator, drained, washed and suctioned to remove excess performance moderator, and then dried with heat to remove any volatiles accompanying the performance moderator. The drying temperature chosen will depend on the nature of the volatiles to be removed. Usually, the time/temperature for effecting drying will be below the conditions for effecting dehydration to remove bound water from the metal oxide in the condensation catalyst to be used in the process of the invention. Normally the drying temperature will be greater than 120°C and below 600°C depending on the thermal stability of the catalyst. The drying time will generally go down as the drying temperature rises and vice versus, and may extend from 5 seconds to about 24 hours.

Alternatively, the performance moderator can be provided to the catalyst at the time of preparing the condensation catalyst in association with one or more metal oxides. For example, one or more metal oxides may be condensed from their respective hydrolyzable monomers to the desired oxides to form oxide powders which can thereafter be blended and compressed with ammonium metatungstate derived substance to form pellets and larger structures of the metal oxide-containing condensation catalyst of this invention. The one or more metal oxides which can be used in association with the condensation catalyst in accordance with this invention can be provided from metal salts which can be heated to form the metal oxide. It is appreciated that the performance moderator can be incorporated into the molecular bonding configuration of the metal oxide-containing condensation catalyst by conventional procedures known in the art.

The condensation catalysts in association with one or more metal oxides prior to the optional treatment of the performance moderator may be prepared in a wide variety of ways. For example, one or more metal oxides may be provided as a partial condensate on a support, such as a silica or alpha, beta or gamma alumina, silicon carbide, and then condensed by heating to effect polymerization to the desired oxide form. The metal oxide(s) may be condensed from hydrolyzable monomers to the desired oxide, indeed, to form an oxide powder which can thereafter be compressed in the presence of an ammonium metatungstate-containing substance to form pellets and larger structures of the metal oxide-containing condensation catalyst of the invention. A blend of the powder and the ammonium metagungstate-containing substance can be made into a shapeable paste which can be extruded and cut into pellets according to conventional procedures. The extrudate may thereafter be fired to cure the ammonium metatungstate-containing substance and fix the structure. The cut extrudate may be blended with a support material such as those characterized above, and the blend fired to fuse the metal oxide-containing catalyst to the support.

In a preferred embodiment of this invention, a high surface area silica or titania can be slurried with an aqueous solution of ammonium metatungstate, extruded, and calcined at a temperature of about 400°C.

A preferred catalyst structure comprises the calcined reaction mixture comprising an ammonium metatungstate in association with a Group IVB metal oxide having a surface area of at least a 140 m²/gm which may or may not be bonded to a support material. The term "support," as used herein and in the claims, means a solid structure which does not adversely affect the catalytic properties of the catalyst and is at least as stable as the catalyst to the reaction medium. The support can function as an amine condensation catalyst, although it may have lower catalytic activity to the reaction. The support may act in concert with the catalyst to moderate the reaction. Some supports may contribute to the selectivity of the reaction. The catalyst structure can comprise from 2 to 60 percent by weight or greater of the support, more preferably from 10 to 50 percent by weight of the support, the remainder being the weight of the metal oxide(s) and ammonium metatungstate-containing substance. Included in the weight of the support is the weight of any binding agent such as phosphates, sulfates, silicates, fluorides, and the like, and any other additive provided to stabilize or otherwise help in the manufacture of the catalyst. The support may be particles as large or larger than the catalyst component and "glued" to the catalytic substance and metal oxide by virtue of a binding medium.

The support may constitute a separate phase in the process of extruding the catalytic structure. In this embodiment, the support forming material, preferably as a paste is blended with a paste of the ammonium metatungstate and one or more metal oxides or a partial condensate thereof. The paste may comprise the oxide forms of the support and the ammonium metatungstate substance, each blended with water, and/or binding agents. The extrudate of the blend is passed through a multiorificed die and chopped into pellets of the desired sizes. The particles may be doughnut shaped, spherical. Then the particles are calcined to dry them and complete any condensation reaction in the support and/or the metal oxide-containing condensation catalyst.

The use of supports for the condensation catalyst to be used in the process of the invention provides a number of significant advantages. When the reaction is effected with the condensation catalyst obtained by calcining a reaction mixture comprising ammonium metatungstate and one or more Group IVB metal oxides as part of a fixed bed in a tubular reactor, the preferred procedure for carrying out the invention, it is desirable to have the catalyst be more stable. When the catalyst is combined with the support, the catalyst has greater stability for the reaction medium, and therefore, it is better able to be used in a fixed bed of a continuous reactor. The supported catalysts suffer from none of the leaching problems that the catalyst per se may have or the problems that are associated with the prior art catalysts, such as acidic phosphorus compounds on silica.

The reactants used in the condensation process of the invention may be ammonia or organic compound containing -NH- and any compound possessing an alcoholic hydroxyl group, subject to the following: the intramolecular condensation of an amino compound produces an amine having a lower molecular weight, and the intermolecular condensation of an amino compound with one or more of another amino compound or a compound containing an alcoholic hydroxyl group produces an amine having a lower, same or higher molecular weight than the reactants.

Illustrative of suitable reactants in effecting the process of the invention, include by way of example:
Ammonia
   - MEA -: monoethanolamine
   - EDA -: ethylenediamine
   - MeEDA -: methylethylenediamine
   - EtEDA -: ethylethylenediamine
   - AEEA -: N-(2-aminoethyl)ethanolamine
   - HEP -: N-(2-hydroxyethyl)piperazine
   - DETA -: diethylenetriamine
   - AEP -: N-(2-aminoethyl)piperazine
   - TAEA -: trisaminoethylamine
   - TETA -: triethylenetetramine
   - TEPA -: tetraethylenepentamine
   - PEHA -: pentaethylenehexamine
TETA Isomers:
   - TAEA -: trisaminoethylamine
   - TETA -: triethylenetetramine
   - DPE -: dipiperazinoethane
   - DAEP -: diaminoethylpiperazine
   - PEEDA -: piperazinoethylethylenediamine
TEPA Isomers:
   - AETAEA -: aminoethyltrisaminoethylamine
   - TEPA -: tetraethylenepentamine
   - AEDPE -: aminoethyldipiperazinoethane
   - AEPEEDA -: aminoethylpiperazinoethylethylenediamine
   - iAEPEEDA -: isoaminoethylpiperazinoethylethylenediamine
   - AEDAEP -: aminoethyldiaminoethylpiperazine
   - BPEA -: bispiperazinoethylamine

The foregoing also can represent the products of the reaction. For example, ammonia and MEA are frequently employed to produce EDA along with a variety of other amines, most of which are set forth above.

Glycol compounds can also be employed in the preparation of amines in accordance with this invention. For purposes of this invention, glycol compounds embrace diols and polyols. Illustrative of suitable glycol compounds include alkylene glycols such as ethylene glycol, propylene glycol, 1,3-propane diol or mixtures thereof.

The process may be effected in the liquid or vapor or supercritical liquid states or mixtures thereof though the actual reaction is believed to occur on the catalyst's solid surface in the absorbed state. In this context, the vapor phase reaction is intended to refer to the general vapor state of the reactants. Though the reaction conditions may range from subatmospheric to superatmospheric conditions, it is desirable to run the reaction from 3.45*10⁵ N/m² to 206.8*10⁵ N/m² gauge (50 psig to 3,000 psig), preferably from 13.8*10⁵ N/m² to 137.8*10⁵ N/m² gauge (200 psig to 2,000 psig).

The temperature of the reaction may be as low as 125°C to 400°C. Preferably, the reaction temperature ranges from 150°C to 350°C, and most preferably from 225°C to 325°C.

The reaction may be effect by the incremental addition of one of the reactants to the other or by the joint addition of the reactants to the catalyst. The preferred process effects the reaction in a continuous manner over a fixed bed of the condensation catalyst obtained by calcining a reaction mixture comprising ammonium metatungstate and one or more Group IVB metal oxides in a tubular reactor. However, the reaction may be carried out by slurrying the catalyst in the reactants or in a batch mode in an autoclave. An inert gas such as nitroge or methane can be used in the reaction process.

The preferred process involves the formation of alkyleneamines from the intermolecular condensation of alkanolamines and alkyleneamines or the intramolecular condensation of alkyleneamines or alkanolamines. Illustrative of such reactions are the following reactant combinations:

| REACTANT | REACTANT | PRODUCTS |
|---|---|---|
| Ammonia | Methanol | Monomethylamine |
| | | Dimethylamine |
| | | Trimethylamine |
| | | |
| Ammonia | MEA | EDA, DETA, AEEA, |
| | | TETA, TEPA, PIP |
| | | |
| Ammonia | AEEA | DETA, PIP |
| | | |
| MEA, Ammonia | EDA | EDA, AEEA, HEP, |
| | | DETA, AEP, TETA, |
| | | TEPA, PEHA, |
| | | TETA Isomers: |
| | | TAEA, TETA, DAEP, |
| | | PEEDA, DPE |
| | | TEPA, TEPA Isomers: |
| | | AETAEA, AEPEEDA, |
| | | AEDAEP, AEDPE, BPEA |
| | | |
| MEA | EDA | AEEA, HEP, DETA, |
| | | AEP, TETA, TEPA, |
| | | PEHA, TETA Isomers: |
| | | TAEA, TETA, DAEP, |
| | | PEEDA, DPE |
| | | TEPA, TEPA Isomers: |
| | | AETAEA, AEPEEDA, |
| | | AEDAEP, AEDPE, BPEA |
| | | |
| EDA | AEEA | HEP, AEP, TETA, |
| | | TEPA, PEHA, |
| | | TETA Isomers: |
| | | TAEA, TETA, DAEP, |
| | | PEEDA, DPE |
| | | TEPA, TEPA Isomers: |
| | | AETAEA, AEPEEDA, |
| | | AEDAEP, AEDPE, BPEA |
| | | |
| DETA | AEEA | TEPA Isomers, |
| | | AEP |
| | | |
| EDA | EDA | DETA, TETA AND |
| | | TEPA Isomers |

The process of the invention provides the ability to generate the manufacture of desirable higher polyalkylene polyamine products such as TETA, TEPA and PEHA without generating large amounts of cyclic alkylenepolyamine products such as PIP, AEP and HEP. The alkyleneamines producers composition of this invention has a TETA + TAEA to PIP + AEP + PEEDA + DAEP + DPE weight ratio of greater than 0.5 and a TETA to TAEA weight ratio of greater than 4.0. The process of this invention provides the potential to control congener distribution, linear to cyclic selectivity and linear to branched selectivity of the higher polyalkylene polyamines.

This invention is further illustrated by certain of the following examples:

### Examples

In the examples set forth below, the catalyst of choice was placed in a tubular reactor having an outside diameter of 2.54 cm (1 inch) and an overall length of 76.2 cm (30 inches). The catalyst portion of the reactor comprised a length of 61.0 cm (24 inches), accommodating 150 cubic centimeters of catalyst. The reactor was made of 316 stainless steel. As used in the examples below, acyclic (N4)/ cyclic (<=N4) refers to the weight ratio of TETA + TAEA to PIP + AEP + PEEDA + DAEP + DPE. The catalysts employed are identified as follows:

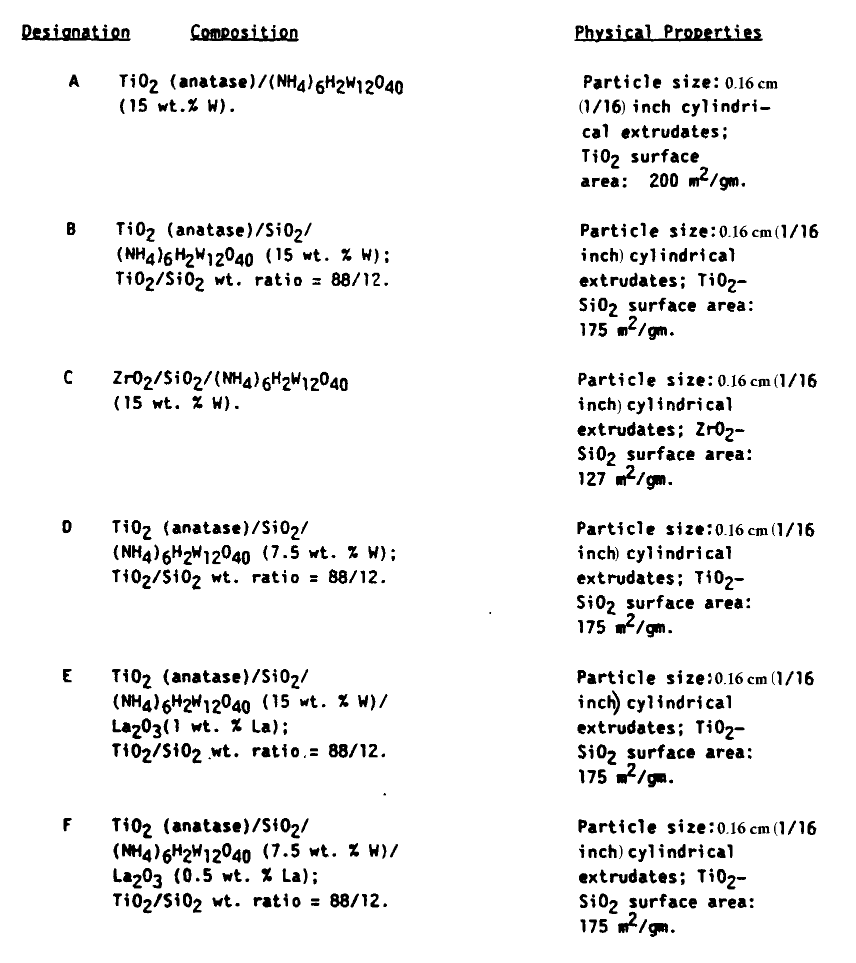

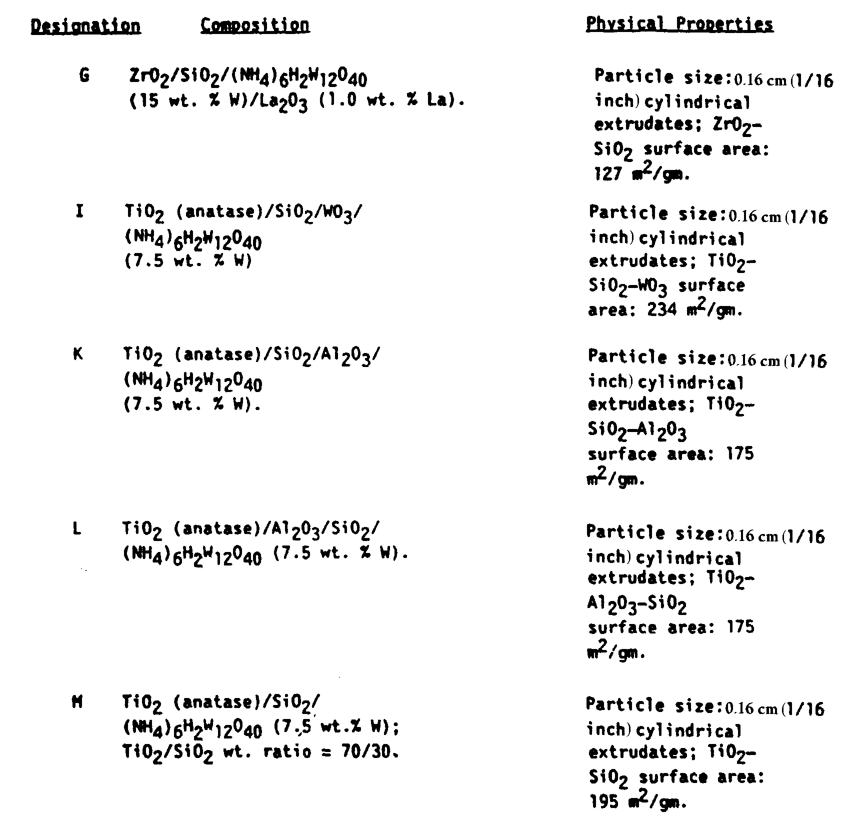

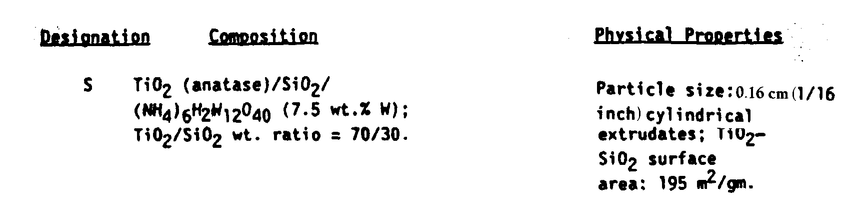

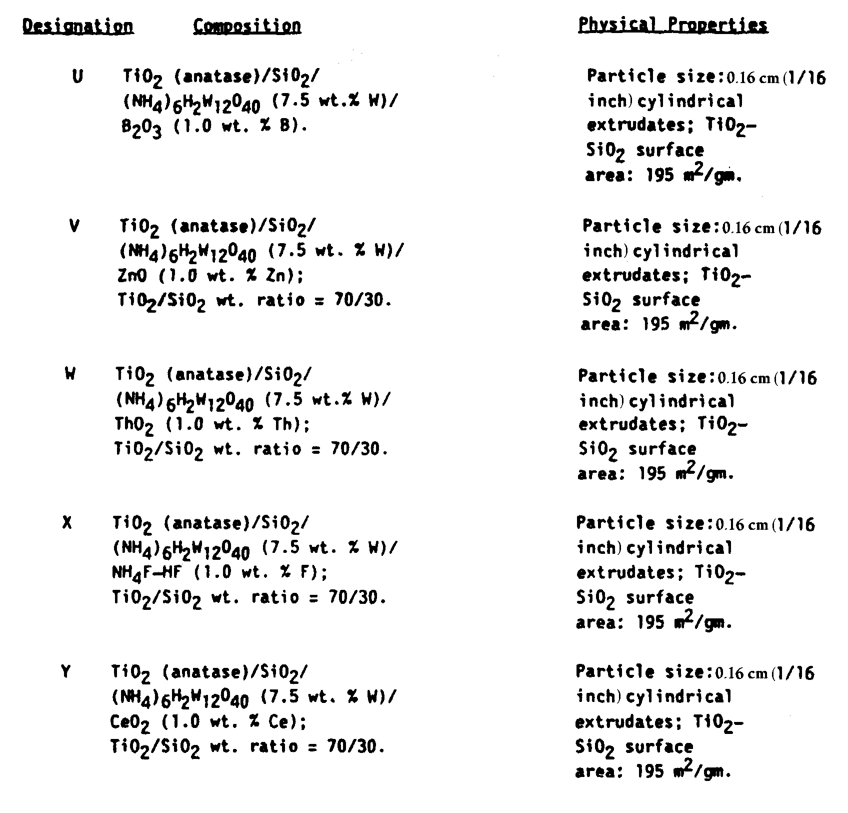

For each run the tubular reaction system was brought up to the designated conditions. The ammonia feed was established first, then the DETA-MEA feed. After a sufficient line out period, a two hour timed run was conducted, then the experiment was run overnight and sampled. The temperature was changed and the above procedure repeated.

The catalysts employed in the examples hereinafter were prepared as follows:
Catalyst A Preparation: Ammonium metatungstate (12.14 grams) was dissolved in water (60 grams) and an aliquot sufficient to wet the TiO₂ support (140 grams) was used. After wetting, the catalyst was calcined at a temperature of 350°C for a period of 1 hour. The impregnation and calcination steps were repeated twice more to give the catalyst.
Catalyst B Preparation: Ammonium metatungstate (12.14 grams) was dissolved in water (48 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst C Preparation: Ammonium metatungstate (12.14 grams) was dissolved in water (48 grams) and an aliquot sufficient to wet the ZrO₂/SiO₂ support (55 grams) was used. After wetting, the catalyst was calcined at a temperature of 350°C for a period of 1 hour. The impregnation and calcination steps were repeated twice more to give the catalyst.
Catalyst D Preparation: Ammonium metatungstate (6.07 grams) was dissolved in water (45 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst E Preparation: Ammonium metatungstate (12.14 grams) and lanthanum nitrate (5.0 grams) were dissolved in water (45 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst F Preparation: Ammonium metatungstate (6.07 grams) and lanthanum nitrate (2.5 grams) were dissolved in water (45 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst G Preparation: Ammonium metatungstate (12.14 grams) and lanthanum nitrate (5.0 grams) were dissolved in water (45 grams) and an aliquot sufficient to wet the ZrO₂/SiO₂ support (55 grams) was used. After wetting, the catalyst was calcined at a temperature of 350°C for a period of 1 hour. The impregnation and calcination steps were repeated twice more to give the catalyst.
Catalyst I Preparation: Ammonium metatungstate (6.07 grams) was dissolved in water (35 grams) and an aliquot sufficient to wet the TiO₂/SiO₂/WO₃ support (55 grams) was used. After wetting, the catalyst was calcined at a temperature of 350°C for a period of 1 hour. The impregnation and calcination steps were repeated twice more to give the catalyst.
Catalyst K Preparation: Ammonium metatungstate (6.07 grams) was dissolved in water (40 grams) and an aliquot sufficient to wet the TiO₂/SiO₂/Al₂O₃ support (55 grams) was used. After wetting, the catalyst was calcined at a temperature of 350°C for a period of 1 hour. The impregnation and calcination steps were repeated twice more to give the catalyst.
Catalyst L Preparation: Ammonium metatungstate (6.07 grams) was dissolved in water (40 grams) and an aliquot sufficient to wet the TiO₂/Al₂O₃/SiO₂ support was used. After wetting, the catalyst was calcined at a temperature of 350°C for a period of 1 hour. The impregnation and calcination steps were repeated twice more to give the catalyst.
Catalyst M Preparation: Ammonium metatungstate (6.07 grams) was dissolved in water (40 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst S Preparation: Ammonium metatungstate (6.07 grams) was dissolved in water (40 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst U Preparation: Ammonium metatungstate (6.07 grams) and boric acid (3.14 grams) were dissolved in water (30 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst V Preparation: Ammonium metatungstate (6.07 grams) and zinc nitrate (1.6 grams) were dissolved in water (30 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst W Preparation: Ammonium metatungstate (6.07 grams) and thorium nitrate (1.31 grams) were dissolved in water (35 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst X Preparation: Ammonium metatungstate (6.07 grams) and ammonium bifluoride (0.82 grams) were dissolved in water (35 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.
Catalyst Y Preparation: Ammonium metatungstate (6.07 grams) and cerium nitrate (1.71 grams) were dissolved in water (35 grams) and an aliquot sufficient to wet the TiO₂/SiO₂ support (55 grams) was used. After wetting, the catalyst was calcined at a temperature of 350°C for a period of 1 hour. The impregnation and calcination steps were repeated twice more to give the catalyst.

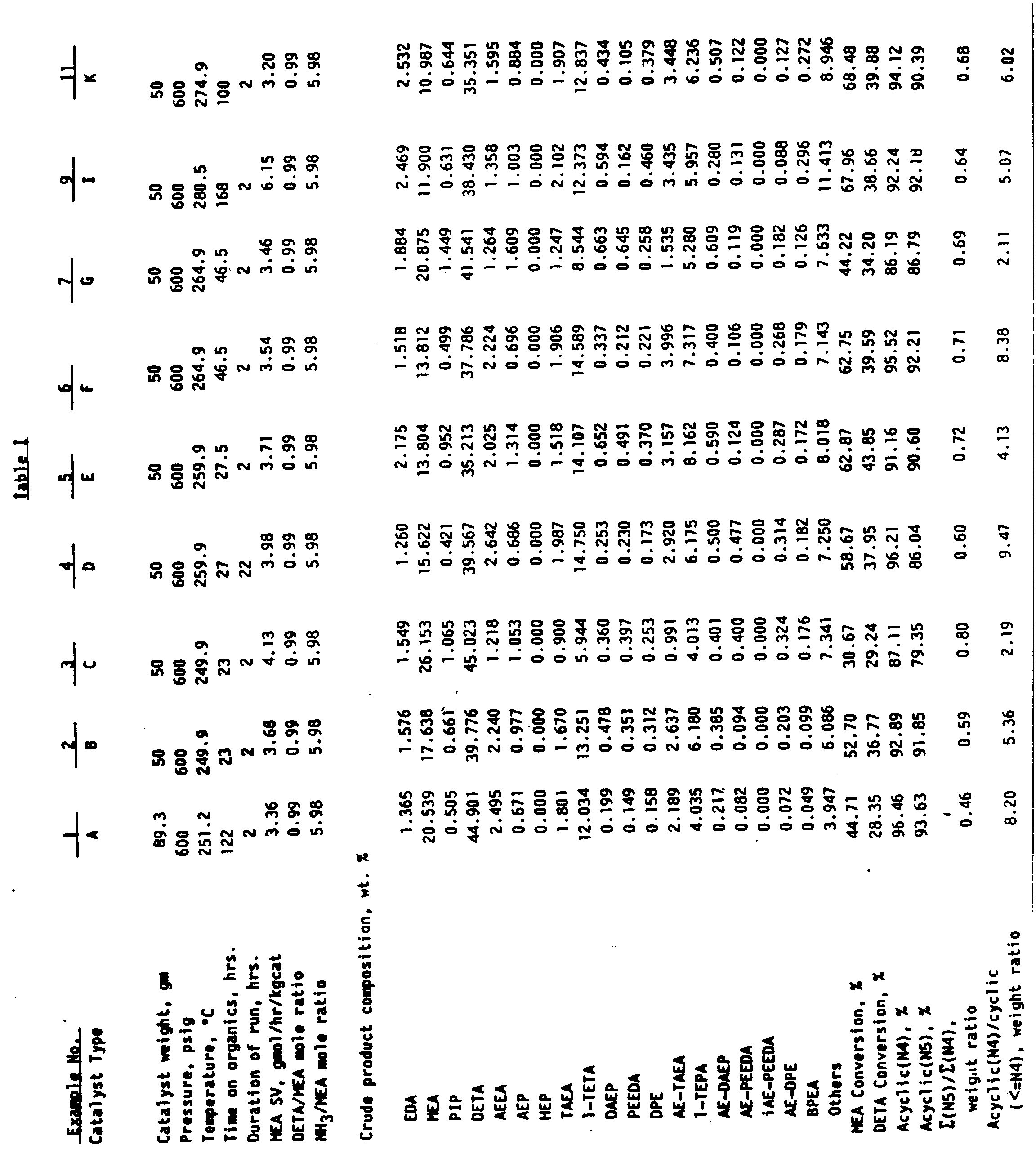

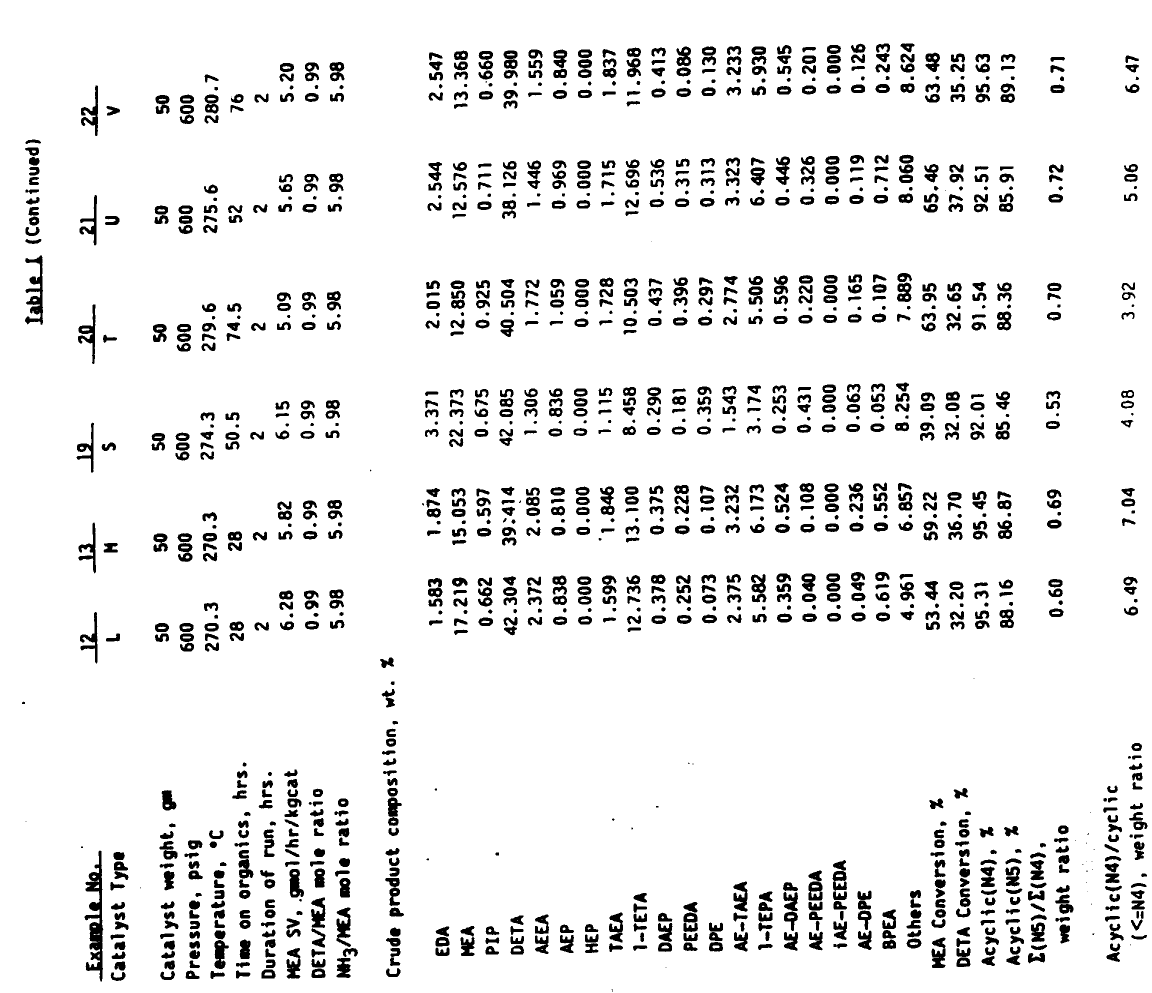

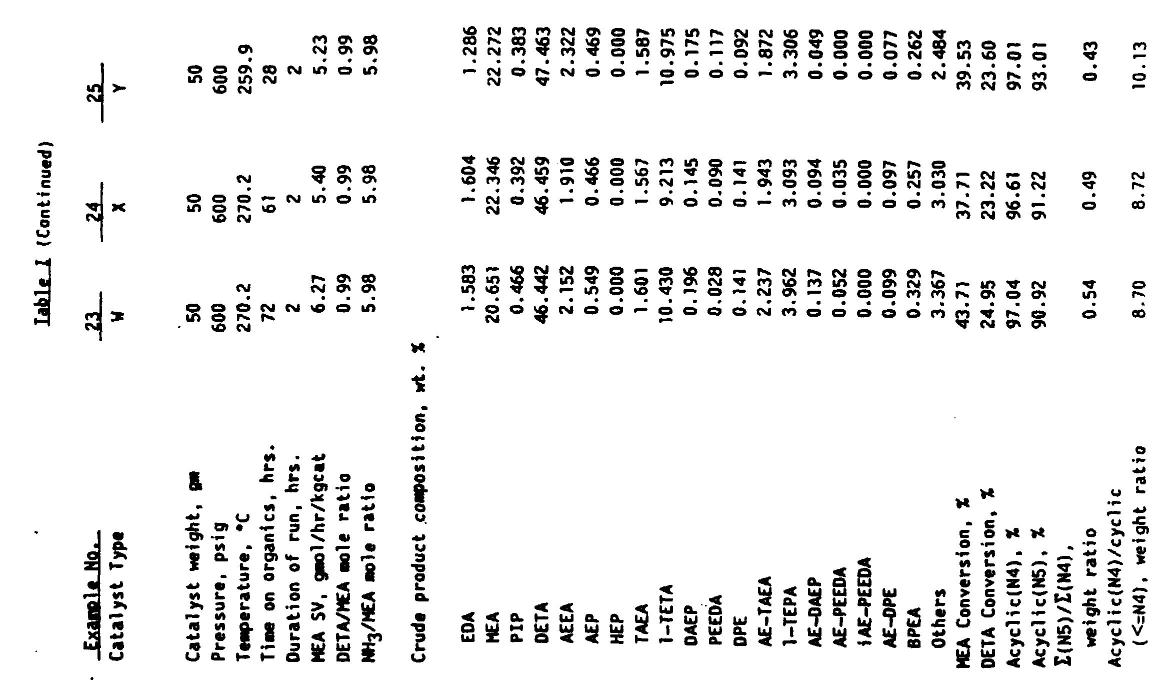

## Claims

1. A process of making polyalkylene polyamines without generating large amounts of cyclics which process comprises condensing, in the absence of hydrogen atoms as a reactant,
(i) an alkanolamine with an alkylene amine or
(ii) an alkanolamine with itself or another alkanolamine, optionally in the presence of ammonia, under condensation conditions in the presence of a condensation catalyst obtained by calcining a reaction mixture comprising ammonium metatungstate and one or more Group IVB metal oxides.

2. The process of claim 1 wherein the catalyst has a surface area of greater than 70 m²/gm.

3. The process of claim 1 or claim 2, wherein the Group IVB metal oxide comprises a high surface area titanium oxide or zirconium oxide.

4. The process of claim 3, wherein the catalyst has a surface area of greater than 140 m²/gm.

5. The process of any one of the claims 1 to 4, wherein the Group IVB metal oxide comprises a mixed oxide of a Group IVB metal and one or more other metals.

6. The process of any one of the claims 1 to 5 wherein the Group IVB metal oxide comprises from 25 wt.% to 90 wt.% of the weight of the catalyst.

7. The process of any one of the claims 1 to 6, which is effected in the liquid phase, vapor phase, supercritical liquid phase or mixtures thereof.

8. The process of any one of the claims 1 to 7, wherein the condensation catalyst further comprises a Group VIII, IB or IIB metal oxide.

9. The process of any one of the claims 1 to 8, wherein the condensation reaction is of said alkanolamine and alkylene amine reactants (i).

10. The process of claim 9 wherein the alkanolamine is monoethanolamine or aminoethylethanolamine.

11. The process of claim 9 or claim 10, wherein the alkylene amine is ethylene diamine or diethylene triamine.

12. The process of any one of claims 9 to 11, wherein the process is effected in the presence of ammonia.

13. The process of claim 9, wherein the alkanolamine is diethanolamine, dihydroxyethylethylenediamine, hydroxyethyl diethylenediamine or hydroxyethyltriethylenetetramine and the alkyleneamine is ethylenediamine or diethylenetriamine.

14. The process of any one of the claims 1 to 8, wherein the condensation reaction is of said alkanolamine reactant (ii) with itself.

15. The process of claim 14, wherein the alkanolamine is monoethanolamine.

## Patentansprüche

1. Verfahren zur Herstellung von Polyalkylenpolyaminen ohne Bildung großer Mengen cyclischer Verbindungen, wobei das Verfahren umfaßt das Kondensieren in Abwesenheit von Wasserstoffatomen als Reaktionspartner von
(i) einem Alkanolamin mit einem Alkylenamin, oder
(ii) einem Alkanolamin mit sich selbst oder einem anderen Alkanolamin,
gegebenenfalls in Gegenwart von Ammoniak unter Kondensationsbedingungen in Gegenwart eines Kondensationskatalysators, erhalten durch Calcinieren eines Reaktionsgemisches, umfassend Ammoniummetawolframat und ein oder mehrere Oxide von Metallen der Gruppe IVB.

2. Verfahren nach Anspruch 1, wobei der Katalysator eine Oberfläche von mehr als 70 m²/g besitzt.

3. Verfahren nach Anspruch 1 oder 2, wobei das Oxid eines Metalls der Gruppe IVB ein Titanoxid oder Zirconiumoxid mit großer Oberfläche umfaßt.

4. Verfahren nach Anspruch 3, wobei der Katalysator eine Oberfläche von mehr als 140 m²/g besitzt.

5. Verfahren nach einem der Ansprüche 1 bis 4, wobei das Oxid eines Metalls der Gruppe IVB ein gemischtes Oxid eines Metalls der Gruppe IVB mit einem oder mehreren anderen Metallen umfaßt.

6. Verfahren nach einem der Ansprüche 1 bis 5, wobei das Oxid eines Metalls der Gruppe IVB 25 bis 90 Gew.-% des Katalysators ausmacht.

7. Verfahren nach einem der Ansprüche 1 bis 6, das in flüssiger Phase, Dampfphase, überkritischer flüssiger Phase oder Gemischen davon durchgeführt wird.

8. Verfahren nach einem der Ansprüche 1 bis 7, wobei der Kondensationskatalysator zusätzlich ein Oxid eines Metalls der Gruppe VIII, IB oder IIB enthält.

9. Verfahren nach einem der Ansprüche 1 bis 8, wobei die Kondensationsreaktion zwischen den Alkanolamin- und Alkylenamin-Reaktionspartnern (i) durchgeführt wird.

10. Verfahren nach Anspruch 9, wobei das Alkanolamin Monoethanolamin oder Aminoethylethanolamin ist.

11. Verfahren nach Anspruch 9 oder 10, wobei das Alkylenamin Ethylendiamin oder Diethylentriamin ist.

12. Verfahren nach einem der Ansprüche 9 bis 11, wobei das Verfahren in Gegenwart von Ammoniak durchgeführt wird.

13. Verfahren nach Anspruch 9, wobei das Alkanolamin Diethanolamin, Dihydroxyethylethylendiamin, Hydroxyethyldiethylendiamin oder Hydroxyethyltriethylentetramin und das Alkylenamin Ethylendiamin oder Diethylentriamin ist.

14. Verfahren nach einem der Ansprüche 1 bis 8, wobei die Kondensationsreaktion zwischen dem Alkanolaminreaktionspartner (ii) selbst durchgeführt wird.

15. Verfahren nach Anspruch 14, wobei das Alkanolamin Monoethanolamin ist.

## Revendications

1. Procédé de préparation de polyalkylènepolyamines sans engendrer de grandes quantités de composés cycliques, procédé qui comprend la condensation, en l'absence d'atomes d'hydrogène comme corps réactionnel,
(i) d'une alcanolamine avec une alkylène-amine, ou
(ii) d'une alcanolamine avec elle-même ou une autre alcanolamine, facultativement en présence d'ammoniac, dans des conditions de condensation en présence d'un catalyseur de condensation obtenu en calcinant un mélange réactionnel comprenant du métatungstate d'ammonium et un ou plusieurs oxydes de métaux du Groupe IVB.

2. Procédé suivant la revendication 1, dans lequel le catalyseur a une surface spécifique supérieure à 70 m²/g.

3. Procédé suivant la revendication 1 ou la revendication 2, dans lequel l'oxyde de métal du Groupe IVB comprend un oxyde de titane ou oxyde de zirconium à grande surface spécifique.

4. Procédé suivant la revendication 3, dans lequel le catalyseur a une surface spécifique supérieure à 140 m²/g.

5. Procédé suivant l'une quelconque des revendications 1 à 4, dans lequel l'oxyde de métal du Groupe IVB comprend un oxyde mixte d'un métal du Groupe IVB et d'un ou plusieurs autres métaux.

6. Procédé suivant l'une quelconque des revendications 1 à 5, dans lequel l'oxyde de métal du Groupe IVB représente 25 % en poids à 90 % en poids du poids du catalyseur.

7. Procédé suivant l'une quelconque des revendications 1 à 6, qui est mis en oeuvre en phase liquide, en phase vapeur, en phase liquide surcritique ou dans des mélanges de ces phases.

8. Procédé suivant l'une quelconque des revendications 1 à 7, dans lequel le catalyseur de condensation comprend en outre un oxyde de métal du Groupe VIII, IB ou IIB.

9. Procédé suivant l'une quelconque des revendications 1 à 8, dans lequel la réaction de condensation est celle des corps réactionnels (i) consistant en l'alcanolamine et l'alkylène-amine.

10. Procédé suivant la revendication 9, dans lequel l'alcanolamine est la mono-éthanolamine ou l'amino-éthyléthanolamine.

11. Procédé suivant la revendication 9 ou la revendication 10, dans lequel l'alkylène-amine est l'éthylènediamine ou la diéthylènetriamine.

12. Procédé suivant l'une quelconque des revendications 9 à 11, qui est mis en oeuvre en présence d'ammoniac.

13. Procédé suivant la revendication 9, dans lequel l'alcanolamine est la diéthanolamine, la dihydroxyéthyléthylènediamine, l'hydroxyéthyldiéthylènediamine ou l'hydroxyéthyltriéthylènetétramine et l'alkylène-amine est l'éthylènediamine ou la diéthylènetriamine.

14. Procédé suivant l'une quelconque des revendications 1 à 8, dans lequel la réaction de condensation est celle de l'alcanolamine servant de corps réactionnel (ii) avec elle-même.

15. Procédé suivant la revendication 14, dans lequel l'alcanolamine est la mono-éthanolamine.
